# FASCICULE DE BREVET EUROPEEN

(11) **EP 1 810 664 B1**
(45) Date de publication et mention de la délivrance du brevet: **02.03.2011**
(21) Numéro de dépôt: 07100526.8
(22) Date de dépôt: 15.01.2007
(51) Int. Cl.: A61K 8/92, A61K 8/86, A61K 8/37, A61Q 19/00, A61Q 19/10

(54) **Composition huileuse colorée**
Gefärbte ölige Zusammensetzung
Coloured oily composition

(30) Priorité: 20.01.2006 FR 0650213
(43) Date de publication de la demande: 25.07.2007
(73) Titulaire: Societe L'Oreal S.A., 75008 Paris (FR)
(72) Inventeur: Tache, Martine, 91170 Viry-Chatillon (FR)
(74) Mandataire: Duvert, Sandra

(56) Documents cités:
- EP-A- 1 576 948

## Description

La présente invention a pour objet une composition cosmétique huileuse colorée comprenant au moins un tensioactif non ionique particulier, et son utilisation dans le domaine cosmétique, notamment comme huile de bain.

Il est connu d'ajouter des huiles, notamment des huiles parfumées, dans l'eau du bain. En effet, on sait que les huiles ont un effet bénéfique sur la peau car elles assurent une protection et une nutrition de la peau, et qu'elles permettent de maintenir la douceur et la flexibilité de la peau en évitant notamment la perte d'eau. Par ailleurs, les huiles de bain parfumées permettent à l'utilisatrice de se parfumer délicatement le corps avec son parfum habituel tout en prenant son bain.

Ces huiles de bain sont de préférence limpides, et elles sont généralement colorées de façon à avoir un aspect visuel plus attrayant. Pour colorer ces huiles, seuls les colorants miscibles aux huiles peuvent être utilisés puisque la composition ne contient que de l'huile. Toutefois, du fait de la législation, tous les colorants ne peuvent être utilisés en cosmétique, et il est parfois impossible de trouver des colorants liposolubles autorisés correspondant à la couleur recherchée. Par ailleurs, s'il est possible de trouver des colorants hydrosolubles autorisés ayant la teinte souhaitée, leur utilisation pose des problèmes de compatibilité puisque ces colorants ne sont pas miscibles aux huiles, et il est impossible d'obtenir une huile de bain limpide avec de tels colorants car le colorant va se déposer sous forme de grumeaux au fond du flacon ou, s'il est en solution aqueuse, il va rester en suspension sous forme de gouttelettes colorées dans l'huile. Le problème se pose plus particulièrement pour certains colorants tels que les colorants rouges car les colorants rouges liposolubles sont interdits par la législation, et ceux autorisés sont hydrosolubles.

Par ailleurs, quand les huiles de bain sont parfumées, le problème de leur coloration est encore plus complexe car il faut que les colorants n'altèrent pas les propriétés des parfums et qu'ils soient compatibles avec le mélange d'huiles et de parfum.

Il subsiste donc le besoin d'une composition huileuse colorée qui puisse contenir un colorant hydrosoluble.

La demanderesse a trouvé de manière surprenante que l'introduction de tensioactifs particuliers permettait d'introduire un colorant hydrosoluble dans une composition huileuse et d'obtenir ainsi une composition huileuse colorée. Les tensioactifs sélectionnés ont permis non seulement d'introduire des colorants hydrosolubles dans les huiles mais aussi d'avoir une huile colorée dont la couleur reste stable dans le temps, et qui, de plus, supporte l'ajout d'un parfum.

Certes, le document EP-A-1576948 décrit des compositions huileuses colorées contenant un tensioactif. Toutefois, ce tensioactif est de préférence anionique comme le montrent tous les exemples décrits, même si certains exemples comportent en plus un tensioactif non ionique, alors que dans la présente demande, les tensioactifs sont choisis parmi des tensioactifs non ioniques spécifiques, non décrits dans ce document. Les exemples comparatifs décrits ci-dessous montrent que seuls ces tensioactifs spécifiques permettent d'atteindre le but de l'invention.

Ainsi, la présente invention a pour objet une composition cosmétique huileuse anhydre, comprenant (1) au moins une huile, (2) au moins un tensioactif non ionique choisi parmi le triisostéarate de PEG-20 glycéryle, le trioléate de PEG-20 sorbitan, le tétraoléate de PEG-30 sorbitan, et leurs mélanges, et (3) un colorant hydrosoluble.

De manière préférée, la composition selon l'invention est exempte de tensioactif anionique.

Le tensioactif utilisé dans la présente invention sert de vecteur d'introduction du colorant hydrosoluble dans l'huile et il permet aussi d'avoir une couleur stable dans le temps. II est en outre utile pour solubiliser le parfum dans l'huile et pour solubiliser l'huile dans l'eau du bain si la composition constitue une huile de bain. Dans ce cas, il est de préférence en quantité suffisante pour émulsionner l'huile de bain dans l'eau, ce qui donne un aspect laiteux à l'eau du bain. Une telle émulsification permet une répartition homogène de l'huile dans l'eau du bain et aussi une répartition homogène du parfum s'il est présent. En outre, une bonne émulsification de l'huile dans l'eau du bain évite l'apparition de gouttelettes d'huile à la surface de l'eau, ce qui est désagréable et peut salir les parois de la baignoire.

La composition selon l'invention est généralement utilisée pour le bain, mais elle peut être utile pour toutes les applications habituelles des huiles dans le domaine cosmétique, notamment des huiles parfumées, et par exemple comme huile pour la peau du corps ou pour les cheveux.

La composition selon l'invention étant une composition cosmétique, donc destinée à une application topique, elle contient un milieu physiologiquement acceptable, c'est-à-dire compatible avec la peau, les muqueuses, les cheveux et le cuir chevelu et les yeux.

On entend par "anhydre" dans le cadre de la présente demande, une composition ne contenant pas d'eau ou une quantité d'eau d'au plus 0,2 % et de préférence d'au plus 0,15% en poids par rapport au poids total de la composition.

### Colorants hydrosolubles

La composition peut contenir tout colorant hydrosoluble approprié en vue d'obtenir la couleur recherchée. Comme colorants, on peut citer par exemple les colorants rouges Cl17200 et Cl14700, les colorants jaunes Cl47000, Cl19140 et C115985, le colorant bleu Cl42090 et le colorant violet Cl60730, et leurs mélanges

On utilise des mélanges de colorants quand on veut obtenir des couleurs impossibles à avoir avec des colorants purs, telles que le rose, ou le vert.

La quantité de colorant(s) hydrosoluble(s) dépend de l'intensité de coloration que l'on veut obtenir et du colorant utilisé. Cette quantité en matière active peut aller par exemple de 0,00001 % à 0,0010 % en poids, de préférence de 0,00001 à 0,0005 % en poids et mieux de 0,00005 à 0,0002 % en poids par rapport au poids total de la composition.

La composition peut éventuellement contenir en outre un ou plusieurs colorants liposolubles, l'addition de tels colorants permettant de compléter ou moduler la couleur obtenue avec le ou les colorants hydrosolubles. La quantité de colorant(s) liposoluble(s) en matière active peut aller par exemple de 0,00001 à 0,0005 % en poids et de préférence de 0,00001 à 0,0001 en poids et mieux de 0,00001 à 0,00004 % en poids par rapport au poids total de la composition.

### Tensioactif non ionique

La composition de l'invention comprend au moins un tensioactif non ionique choisi parmi le triisostéarate de PEG-20 glycéryle (nom INCl : PEG-20 glyceryl triisostearate), le trioléate de PEG-20 sorbitan (ou PEG-20 sorbitan trioleate) (nom INCl : Polysorbate 85), le tétraoléate de PEG-30 sorbitan (nom INCl : PEG-30 sorbitan tetraoleate), et leurs mélanges. II peut y avoir un seul de ces tensioactifs ou un mélange de ces tensioactifs. Toutefois, la composition est de préférence exempte de tensioactifs autres que ceux indiqués ci-dessus.

La quantité de tensioactif(s) dépend de la présence ou non de parfum, et si la composition en contient, cette quantité dépend aussi du taux de parfum. Cette quantité peut aller par exemple de 0,5 à 20 % en poids, de préférence de 1 à 20 % en poids, mieux de 2 à 15 % en poids et encore mieux de 2 à 10 % en poids par rapport au poids total de la composition.

Comme triisostéarate de PEG-20 glycéryle, on peut citer par exemple celui commercialisé par la société Nihon Emulsion sous les noms EMALEX GWIS-305 et EMALEX GWIS-320EX.

Comme trioléate de PEG-20 sorbitan, on peut citer par exemple celui commercialisé sous la dénomination Rheodol TW-0320V par la société Kao ou celui commercialisé sous la dénomination Tween 85 V par la société Uniqema ou celui commercialisé sous la dénomination NIKKOL TO-30 V par la société Nikkol.

Selon un mode préféré de réalisation de l'invention, le tensioactif non ionique utilisé est choisi parmi le triisostéarate de PEG-20 glycéryle, le trioléate de PEG-20 sorbitan et leurs mélanges.

### Huiles

La composition selon l'invention contient au moins une huile, notamment une huile cosmétique qui peut être choisie parmi les hydrocarbures substantiellement linéaires ou ramifiés, d'origine minérale ou synthétique ; les huiles hydrocarbonées ; les huiles de synthèse ; les éthers ; les alcools gras ; les huiles fluorées ; les huiles de silicone; et leurs mélanges.

Parmi les huiles pouvant rentrer dans la composition de la phase huileuse, on peut notamment citer :
- les hydrocarbures substantiellement linéaires ou ramifiés, d'origine minérale ou synthétique, notamment les huiles minérales telles que l'huile minérale obtenue du pétrole, l'huile de vaseline telle que celles commercialisées sous les références Marcol 82, Marcol 52, Marcol 172 et Primol 352 par la société Esso, les huiles de paraffine, volatiles ou non ; les polydécènes ; l'isohexadecane ; l'isododecane ; le polyisobutène hydrogéné tel que l'huile de Parléam^{®} ;
- les huiles hydrocarbonées d'origine animale telles que le perhydrosqualène ;
- les huiles hydrocarbonées d'origine végétale telles que l'huile d'amande douce, l'huile de cassis, l'huile de bourrache, l'huile de chanvre, l'huile d'avocat, l'huile de ricin, l'huile de coriandre, l'huile d'olive, l'huile de jojoba, l'huile de sésame, l'huile d'arachide, l'huile de pépins de raisin, l'huile de colza, l'huile de coprah, l'huile de noisette, le beurre de karité, l'huile de palme, l'huile de noyau d'abricot, l'huile de calophyllum, l'huile de son de riz, l'huile de germes de maïs, l'huile de germes de blé, l'huile de soja, l'huile de tournesol, l'huile d'onagre, l'huile de carthame, l'huile de passiflore, l'huile de seigle, les triglycérides des acides caprylique/caprique comme ceux vendus par la société Stearineries Dubois ou ceux vendus sous les dénominations Miglyol 810, 812 et 818 par la société Dynamit Nobel ;
- les huiles de synthèse, telles que l'huile de purcellin ; les esters d'acide gras de formule R¹COOR² dans laquelle R¹ représente le reste d'un acide gras comportant de 8 à 29 atomes de carbone, et R² représente une chaîne hydrocarbonée, ramifiée ou non, contenant de 3 à 30 atomes de carbone, comme le myristate de butyle, le myristate d'isopropyle, le myristate de cétyle, le palmitate d'isopropyle, le palmitate d'ethyl-2 hexyle (ou palmitate d'octyle), l'adipate d'isopropyle, l'adipate d'éthylhéxyle, le stéarate de butyle, le stéarate d'héxadécyle, le stéarate d'isopropyle, le stéarate d'octyle, le stéarate d'isocétyle, l'oléate de décyle, le laurate d'héxyle, le dicaprylate de propylène glycol et les esters dérivés d'acide lanolique tels que le lanolate d'isopropyle, le lanolate d'isocétyle ;
- les éthers de formule R¹OR² dans laquelle R¹ représente le reste d'un acide gras comportant de 8 à 29 atomes de carbone, et R² représente une chaîne hydrocarbonée, ramifiée ou non, contenant de 3 à 30 atomes de carbone, tels que l'éther dicaprylique (nom CTFA : Dicaprylyl ether) ; les benzoates d'alcools gras en C₁₂-C₁₅ (Finsolv TN de FINETEX) ;
- les alcools gras comportant de 8 à 26 atomes de carbone, tels que les alcools laurique, cétylique, myristique, stéarylique, palmitique, oléique, linoléique, le 2-octyldodécanol, et leurs mélanges tels que l'alcool cétéarylique (mélange d'alcool cétylique et d'alcool stéarylique) ;
- les huiles fluorées et perfluorées comme par exemple le méthyl perfluorobutyléther ;
- les huiles de silicone, volatiles ou non, comme les polyméthylsiloxanes (PDMS) volatiles ou non à chaîne siliconée linéaire ou cyclique, liquides ou pâteux à température ambiante, notamment les silicones volatiles cycliques comme les cyclopolydiméthylsiloxanes (cyclométhicones) telles que la cyclohexasiloxane et la cylcopentasiloxane ; les polydiméthylsiloxanes comportant des groupements alkyle, alcoxy ou phényle, pendant ou en bout de chaîne siliconée, groupements ayant de 2 à 24 atomes de carbone ; les silicones phénylées comme les phényltriméthicones, les phényldiméthicones, les phényltriméthylsiloxydiphénylsiloxanes, les diphényl-diméthicones, les diphénylméthyldiphényl trisiloxanes, les 2-phényléthyltriméthyl-siloxysilicates, les polyméthyl-phénylsiloxanes.

On entend par « huile hydrocarbonée » dans la liste des huiles citées ci-dessus, toute huile comportant majoritairement des atomes de carbone et d'hydrogène, et éventuellement des groupements ester, éther, fluoré, acide carboxylique et/ou alcool.

On peut utiliser une huile ou un mélange de plusieurs des huiles indiquées ci-dessus. Selon un mode préféré de réalisation de l'invention, la composition contient au moins une huile choisie parmi les huiles minérales, les esters d'acide gras, et leurs mélanges.

Selon un mode préféré de réalisation de l'invention, la composition selon l'invention contient avantageusement au moins un ester d'acide gras, et notamment au moins du palmitate d'éthyl hexyle. De manière préférée, on utilise un mélange d'huile de vaseline et d'ester dont la palmitate d'éthyl hexyle.

Par ailleurs, l'ajout d'une huile perfluorée ou d'une huile de silicone volatile peut être intéressante pour apporter un effet frais, notamment dans les huiles pour le corps.

La quantité totale d'huiles peut aller par exemple de 80 à 99,5 % en poids, de préférence de 85 à 99 % en poids, et mieux de 90 à 98 % en poids par rapport au poids total de la composition.

### Additifs

La composition selon l'invention peut comprendre en outre les adjuvants classiquement mis en oeuvre dans le domaine cosmétique. Elle peut contenir en particulier au moins un adjuvant choisi parmi les antioxydants, les parfums, les filtres U.V., les actifs lipophiles, les conservateurs, les extraits végétaux, et tout autre ingrédient habituellement utilisé en cosmétique, et leurs mélanges. Elle peut contenir aussi un peu d'éthanol en petites quantités, des traces de cet alcool pouvant être présent dans le parfum ajouté à la composition.

Les quantités des différents constituants des compositions selon l'invention sont celles classiquement utilisées dans le domaine considéré. Bien entendu, ces adjuvants doivent être de nature et utilisés en quantité telle qu'ils ne perturbent pas la composition selon l'invention c'est-à-dire qu'elle reste limpide et colorée. La quantité de ces adjuvants peut aller par exemple de 0,0001 à 10 % en poids par rapport au poids total de la composition.

Selon un mode préféré de réalisation de l'invention, la composition contient un parfum. On entend par « parfum » aussi bien une substance odorante isolée qu'un mélange de substances odorantes. On peut utiliser comme parfums, les mélanges de substances odorantes disponibles sur le marché des parfums, ceci afin que l'huile de bain ait la même odeur que le parfum habituellement utilisé par la personne prenant un bain, ou toutes les substances odorantes isolées ou en mélange.

Comme substance odorante, on peut utiliser les parfums et les arômes d'origine naturelle ou synthétique et leurs mélanges. Comme parfums et arômes d'origine naturelle, on peut citer par exemple les extraits de fleurs (lis, lavande, rose, jasmin, ilang-ilang), de tiges et de feuilles (patchouli, géranium, petit-grain), de fruits (coriandre, anis, cumin, genièvre), d'écorces de fruits (bergamote, citron, orange), de racines (angélique, céleri, cardamome, iris, acore), de bois (bois de pin, santal, gaïac, cèdre rose), d'herbes et de graminées (estragon, lemon grass, sauge, thym), d'aiguilles et de branches (épicéa, sapin, pin, pin nain), de résines et de baumes (galbanum, élémi, benjoin, myrrhe, oliban, opopanax).

Comme substance odorante d'origine synthétique, on peut citer par exemple les composés du type ester, éther, aldéhyde, cétone, alcool aromatique et hydrocarbure :
- Comme esters, on peut citer en particulier l'acétate de benzyle, le benzoate de benzyle, l'isobutyrate de phénoxyéthyle, l'acétate de p-tert-butylcyclohexyle, l'acétate de citronellyle, le formiate de citronellyle, l'acétate de géranyle, l'acétate de linalyle, l'acétate de diméthyl-benzylcarbinyle, l'acétate de phényléthyle, le benzoate de linalyle, le formiate de benzyle, le glycinate d'éthylméthylphényle, le propionate d'alkylcyclohexyle, le propionate de styralyle et le salicylate de benzyle.
- Comme éthers, on peut citer le benzyléthyléther.
- Comme aldéhydes, on peut citer par exemple les alcanals linéaires comportant de 8 à 18 atomes de carbone, le citral, le citronellal, le citronellyloxyacétaldéhyde, le cyclamènaldéhyde, l'hydroxycitronellal, le lilial et le bourgeonal.
- Comme cétones, on peut citer par exemple les ionones comme l'alpha-isométhylionone, et la méthylcédrylcétone.
- Comme alcool aromatiques et notamment terpéniques, on peut citer l'anéthol, le citronellol, l'eugénol, l'isoeugénol, le géraniol, le linalol, le phényléthylalcool et le terpinéol.
- Comme hydrocarbures, on peut citer notamment les terpènes.

Par ailleurs, on peut aussi utiliser des huiles essentielles, composants d'arômes, comme par exemple les essences de sauge, de camomille, de girofle, de mélisse, de menthe, de feuilles de cannelier, de fleurs de tilleul, de genièvre, de vétiver, d'olibian, de galbanum, de labolanum et de lavandin.

Selon un mode préféré de réalisation, on utilise un mélange de différentes substances odorantes qui engendrent en commun une note parfumante plaisante pour l'utilisateur.

La quantité de parfum varie selon l'intensité de parfumage souhaitée et le but d'utilisation de l'huile. Cette quantité peut aller par exemple de 0,01 à 15 % en poids, de préférence de 0,05 à 10 % en poids et mieux de 0,5 à 8 % en poids par rapport au poids total de la composition. Dans une huile de bain, la quantité de parfum peut aller par exemple de 0,5 à 10 % et de préférence de 1 à 5 % en poids par rapport au poids total de la composition.

La présente invention a aussi pour objet une composition cosmétique huileuse anhydre, comprenant (1) au moins une huile, (2) au moins un tensioactif non ionique choisi parmi le triisostéarate de PEG-20 glycéryle, le trioléate de PEG-20 sorbitan, le tétraoléate de PEG-30 sorbitan, et leurs mélanges, (3) un colorant hydrosoluble et (4) un parfum.

Quand la composition contient un parfum, il peut être intéressant d'ajouter une petite quantité de filtre U.V. qui va protéger le parfum de la dégradation due à l'effet de la lumière, surtout quand la composition est mise dans un flacon transparent.

Les compositions selon l'invention sont appropriées pour toute utilisation cosmétique d'huile colorée. Elles peuvent constituer notamment des huiles de bain colorées, éventuellement parfumées. Elles peuvent aussi éventuellement constituer une huile pour le soin du corps ou le soin des cheveux, ou une huile à utiliser après la douche ou après le bain.

Un autre objet de l'invention consiste en l'utilisation cosmétique d'une composition cosmétique telle que définie ci-dessus, comme huile de bain, huile de soin du corps ou des cheveux, une huile à utiliser après la douche ou après le bain.

De manière préférée, la composition selon l'invention est préparée par mélange préalable du tensioactif et des colorants avant incorporation éventuelle du parfum, puis ajout des huiles.

### Exemple 1 selon l'invention

| *Phase A* | | |
|---|---|---|
| PEG-20 glyceryl triisostearate | 4 % | |
| Colorant rouge Cl 17200 | 0,00009 % | |
| Colorant jaune Cl 19140 | 0,000068 % | |
| | | |

| *Phase B* | | |
|---|---|---|
| Parfum | 5 % | |
| Ethylhexyl methoxycinnamate | 1 % | |
| | | |

| *Phase C* | | |
|---|---|---|
| Huile de vaseline | qsp 100 | % |
| Palmitate d'éthyl hexyle | 38 | % |

### Mode de préparation :

- on a préparé la phase A à la température ambiante sous agitateur magnétique pour solubiliser les poudres de colorants dans le tensioactif,
- on a préparé la phase B en solubilisant le filtre dans le parfum à froid,
- on a mélangé les huiles de la phase C,
- on a versé la phase A dans la phase B sous agitation, et on y a ajouté la phase C.

On a obtenu une huile limpide de couleur rose abricot, stable à 2 mois à température ambiante aussi bien qu'à 37°C, à 45°C et à 4°C.

Une petite quantité de cette huile parfumée versée dans l'eau chaude de bain donne une eau laiteuse blanche parfumée avec un fini doux sur la peau.

Cette huile peut également être appliquée directement sur le corps.

### Exemple comparatif 1

| *Phase A* | | |
|---|---|---|
| PEG-20 sorbitan isostearate (NikkoL TI-10V) | 4 % | |
| Colorant rouge Cl 17200 | 0,00009 % | |
| Colorant jaune Cl 19140 | 0,000068 % | |
| | | |

| *Phase B* | | |
|---|---|---|
| Parfum | 5 % | |
| Ethylhexyl methoxycinnamate | 1 % | |
| | | |

| *Phase C* | | |
|---|---|---|
| Huile de vaseline | qsp 100 | % |
| Palmitate d'éthyl hexyle | 38 | % |

Le mode opératoire est le même que dans l'exemple 1. L'huile obtenue n'est plus homogène et les colorants décantent.

### Exemple comparatif 2

| *Phase A* | | |
|---|---|---|
| PEG-30 Dipolyhydroxystearate (Arlacel P135) | 4 % | |
| Colorant rouge Cl 17200 | 0,00009 % | |
| Colorant jaune Cl 19140 | 0,000068 % | |
| | | |

| *Phase B* | | |
|---|---|---|
| Parfum | 5 % | |
| Ethylhexyl methoxycinnamate | 1 % | |

| *Phase C* | | |
|---|---|---|
| Huile de vaseline | qsp 100 | % |
| Palmitate d'éthyl hexyle | 38 | % |

Le mode opératoire est le même que dans l'exemple 1. L'huile obtenue n'est plus homogène, mais trouble, et le tensioactif ne se mélange pas à l'huile.

Les exemples comparatifs 1 et 2 montrent que des tensioactifs oxyéthylénés autres que ceux revendiqués ne permettent pas d'atteindre le but de l'invention.

### Exemple comparatif 3

| *Phase A* | | |
|---|---|---|
| Cocamide MEA (Alkamide C-212) | 4 % | |
| Colorant rouge Cl 17200 | 0,00009 % | |
| Colorant jaune Cl 19140 | 0,000068 % | |
| | | |

| *Phase B* | | |
|---|---|---|
| Parfum | 5 % | |
| Ethylhexyl methoxycinnamate | 1 % | |
| | | |

| *Phase C* | | |
|---|---|---|
| Huile de vaseline | qsp 100 | % |
| Palmitate d'éthyl hexyle | 38 | % |

Le mode opératoire est le même que dans l'exemple 1. L'huile obtenue n'est plus homogène, et le tensioactif ne se mélange pas à l'huile mais de dépose au fond du flacon avec le colorant.

Cet exemple comparatifs montre que le Cocamide MEA cité parmi les tensioactifs préférés dans le document EP-A-1,576,948 ne permet pas d'atteindre le but de l'invention et d'obtenir une huile colorée homogène.

### Exemple 2 selon l'invention

| *Phase A* | | |
|---|---|---|
| Polysorbate 85 | 4 % | |
| Colorant rouge Cl 17200 | 0,00007 % | |
| Colorant jaune Cl 47000 | 0,00005 % | |
| | | |

| *Phase B* | | |
|---|---|---|
| Parfum | 5 % | |
| Ethylhexyl salicylate | 0,5 % | |
| | | |

| *Phase C* | | |
|---|---|---|
| Huile de vaseline | qsp 100 | % |
| Palmitate d'éthyl hexyle | 40 | % |

On a préparé cet exemple selon le même mode opératoire que l'exemple 1. On a obtenu une huile limpide de couleur rose abricot, stable à 2 mois à température ambiante aussi bien qu'à 37°C, à 45°C et à 4°C.

### Exemple comparatif 4

On a fait l'exemple 2 selon l'invention en remplaçant le Polysorbate 85 (PEG-20 sorbitan trioleate) par du trioléate de sorbitan (Sorbitan trioleate) (NIKKOL SO-30 V de la société Nikko). La coloration rosée obtenue au temps zéro s'est rapidement modifiée jusqu'à devenir jaune au bout de quelques jours, le colorant rose se déposant au fond du flacon. Cet exemple montre que les tensioactifs analogues à ceux utilisés dans la présente invention mais dépourvus de groupes oxyéthylénés ne permettent pas d'obtenir une huile colorée dont la couleur reste stable dans le temps.

## Revendications

1. Composition cosmétique anhydre, comprenant (1) au moins une huile, (2) au moins un tensioactif non ionique choisi parmi le triisostéarate de PEG-20 glycéryle, le trioléate de PEG-20 sorbitan, le tétraoléate de PEG-30 sorbitan, et leurs mélanges, et (3) un colorant hydrosoluble.

2. Composition selon la revendication 1, **caractérisée en ce qu'**elle est exempte de tensioactif anionique.

3. Composition selon l'une quelconque des revendications précédentes, **caractérisée en ce que** la quantité de colorant hydrosoluble va de 0,00001 % à 0,0010 % en poids par rapport au poids total de la composition.

4. Composition selon la revendication 1 ou 2, **caractérisée en ce que** le colorant hydrosoluble est choisi parmi le CI17200, le CI14700, le CI19140, le CI15985, le C142090, le CI60730, et leurs mélanges.

5. Composition selon l'une quelconque des revendications précédentes, **caractérisée en ce que** la quantité de tensioactif va de 0,5 à 20 % en poids par rapport au poids total de la composition.

6. Composition selon l'une quelconque des revendications précédentes, **caractérisée en ce que** l'huile est choisie parmi les huiles minérales, les esters d'acide gras et leurs mélanges.

7. Composition selon l'une quelconque des revendications précédentes, **caractérisée en ce qu'**elle contient du palmitate d'éthyl hexyle.

8. Composition selon l'une quelconque des revendications précédentes, **caractérisée en ce qu'**elle contient une quantité totale d'huiles allant de 80 à 99,5 % en poids par rapport au poids total de la composition.

9. Composition selon l'une quelconque des revendications précédentes, **caractérisée en ce qu'**elle contient en outre un parfum.

10. Composition cosmétique huileuse anhydre, comprenant (1) au moins une huile, (2) au moins un tensioactif non ionique choisi parmi le triisostéarate de PEG-20 glycéryle, le trioléate de PEG-20 sorbitan, le tétraoléate de PEG-30 sorbitan, et leurs mélanges, (3) un colorant hydrosoluble et (4) un parfum.

11. Composition selon l'une quelconque des revendications précédentes, **caractérisée en ce qu'**elle constitue une huile de bain, une huile de soin du corps ou des cheveux, une huile à utiliser après la douche ou après le bain.

12. Utilisation cosmétique d'une composition cosmétique selon l'une quelconque des revendications 1 à 10, comme huile de bain ou comme huile de soin du corps ou des cheveux.

## Claims

1. Anhydrous cosmetic composition, comprising (1) at least one oil, (2) at least one non-ionic surfactant chosen from PEG-20 glyceryl triisostearate, PEG-20 sorbitan trioleate, PEG-30 sorbitan tetraoleate, and mixtures thereof, and (3) a water-soluble dye.

2. Composition according to Claim 1, **characterized in that** it is free of anionic surfactant.

3. Composition according to either one of the preceding claims, **characterized in that** the amount of water-soluble dye ranges from 0.00001% to 0.0010% by weight relative to the total weight of the composition.

4. Composition according to Claim 1 or 2, **characterized in that** the water-soluble dye is chosen from CI17200, CI14700, CI19140, CI15985, CI42090, CI60730, and mixtures thereof.

5. Composition according to any one of the preceding claims, **characterized in that** the amount of surfactant ranges from 0.5% to 20% by weight relative to the total weight of the composition.

6. Composition according to any one of the preceding claims, **characterized in that** the oil is chosen from mineral oils, fatty acid esters and mixtures thereof.

7. Composition according to any one of the preceding claims, **characterized in that** it contains ethylhexyl palmitate.

8. Composition according to any one of the preceding claims, **characterized in that** it contains a total amount of oils ranging from 80% to 99.5% by weight relative to the total weight of the composition.

9. Composition according to any one of the preceding claims, **characterized in that** it also contains a fragrance.

10. Anhydrous oily cosmetic composition, comprising (1) at least one oil, (2) at least one non-ionic surfactant chosen from PEG-20 glyceryl triisostearate, PEG-20 sorbitan trioleate, PEG-30 sorbitan tetraoleate, and mixtures thereof, (3) a water-soluble dye and (4) a fragrance.

11. Composition according to any one of the preceding claims, **characterized in that** it constitutes a bath oil, a body care oil or hair care oil, an oil to be used after showering or after bathing.

12. Cosmetic use of a cosmetic composition according to any one of Claims 1 to 10 as bath oil or as body care oil or hair care oil.

## Patentansprüche

1. Wasserfreie, kosmetische Zusammensetzung, die (1) mindestens ein Öl, (2) mindestens einen nichtionischen grenzflächenaktiven Stoff, der unter PEG-20-gylceryltriisostearat, PEG-20-sorbitantrioleat, PEG-30-sorbitantetraoleat und deren Gemischen ausgewählt ist, und (3) einen wasserlöslichen Farbstoff enthält.

2. Zusammensetzung nach Anspruch 1, **dadurch gekennzeichnet, dass** sie keinen anionischen grenzflächenaktiven Stoff enthält.

3. Zusammensetzung nach einem der vorhergehenden Ansprüche, **dadurch gekennzeichnet, dass** der Mengenanteil des wasserlöslichen Farbstoffes im Bereich von 0,00001 bis 0,0010 Gew.-%, bezogen auf das Gesamtgewicht der Zusammensetzung, liegt.

4. Zusammensetzung nach Anspruch 1 oder 2, **dadurch gekennzeichnet, dass** der wasserlösliche Farbstoff unter CI17200, CI14700, CI19140, CI15985, CI42090, CI60730 und deren Gemischen ausgewählt ist.

5. Zusammensetzung nach einem der vorhergehenden Ansprüche, **dadurch gekennzeichnet, dass** der Mengenanteil des grenzflächenaktiven Stoffes im Bereich von 0,5 bis 20 Gew.-%, bezogen auf das Gesamtgewicht der Zusammensetzung, liegt.

6. Zusammensetzung nach einem der vorhergehenden Ansprüche, **dadurch gekennzeichnet, dass** das Öl unter den Mineralölen, Fettsäureestern und deren Gemischen ausgewählt ist.

7. Zusammensetzung nach einem der vorhergehenden Ansprüche, **dadurch gekennzeichnet, dass** sie Ethylhexylpalmitat enthält.

8. Zusammensetzung nach einem der vorhergehenden Ansprüche, **dadurch gekennzeichnet, dass** sie eine Gesamtmenge an Ölen von 80 bis 99,5Gew.-%, bezogen auf das Gesamtgewicht der Zusammensetzung, enthält.

9. Zusammensetzung nach einem der vorhergehenden Ansprüche, **dadurch gekennzeichnet, dass** sie ferner ein Parfum enthält.

10. Wasserfreie, ölige, kosmetische Zusammensetzung, die (1) mindestens ein Öl, (2) mindestens einen nichtionischen grenzflächenaktiven Stoff, der unter PEG-20-gylceryltriisostearat, PEG-20-sorbitantrioleat, PEG-30-sorbitantetraoleat und deren Gemischen ausgewählt ist, (3) einen wasserlöslichen Farbstoff und (4) ein Parfum enthält.

11. Zusammensetzung nach einem der vorhergehenden Ansprüche, **dadurch gekennzeichnet, dass** es sich um ein Ölbad, ein Öl für die Pflege des Körpers oder der Haare, ein nach dem Bad oder der Dusche anzuwendendes Öl handelt.

12. Kosmetische Verwendung einer kosmetischen Zusammensetzung nach einem der Ansprüche q bis 10 als Ölbad oder Öl für die Pflege des Körpers oder der Haare.
